# EUROPEAN PATENT APPLICATION

(11) **EP 3 925 541 A1**
(43) Date of publication of application: **22.12.2021**
(21) Application number: 20180792.2
(22) Date of filing: 18.06.2020
(51) Int. Cl.: A61B 6/06, A61B 6/10, A61B 6/00, G21F 3/02

(54) **REDUCING PHASE-CONTRAST OR DARK-FIELD IMAGING ARTEFACTS**

(71) Applicant: Koninklijke Philips N.V., 5656 AG Eindhoven (NL)
(72) Inventor: KOEHLER, Thomas, 5656 AE Eindhoven (NL); YAROSHENKO, Andre, 5656 AE Eindhoven (NL); VOGTMEIER, GEREON, 5656 AE Eindhoven (NL)
(74) Representative: Philips Intellectual Property & Standards

(57) **Abstract**

The present invention relates to a method (100) for reducing image artefacts in dark-field and/or phase-contrast X-ray imaging of a subject. The method comprises positioning (101) the subject to be imaged between an imaging detector and an X-ray source of an X-ray imaging apparatus, and positioning (102) an X-ray attenuating material between the imaging detector and the X-ray source such that direct exposure of the detector by radiation emitted by the source during imaging of the subject is reduced or prevented. Further aspects of the invention relate to a garment suitable for use in such method, an attachment for attaching to clothing to form such garment, and a cushion suitable for use in such method.

## Description

### FIELD OF THE INVENTION

The present invention relates to the field of X-ray dark-field and phase-contrast imaging, and, more specifically, the invention relates to methods and devices for avoiding, preventing and/or reducing image artefacts in dark-field imaging and phase-contrast imaging.

### BACKGROUND OF THE INVENTION

X-ray dark-field imaging has the remarkable advantage of being able to reveal information about structures at a scale below the limits of spatial resolution of the imaging system that is used. This property can be used in many applications, such as, for example, in medical diagnosis of pulmonary disorders. It has been found that dark-field imaging can significantly increase the diagnostic accuracy in detecting such pulmonary disorders, e.g. when compared to conventional X-ray attenuation imaging. This can be understood as a result of the sensitivity of dark-field imaging to small-angle scattering of radiation by particularly small structures, e.g. specifically by the alveoli.

Alveoli are small structures in the lung that are responsible for gas exchange with the blood. Thus, these structures are responsible for providing the primary function of the lungs. Therefore, it will be appreciated that any condition that adversely affects the alveoli potentially has severe consequences, e.g. by threatening this vital function. However, the small scale of these alveoli makes the detection of abnormalities, e.g. of adverse conditions, by conventional imaging modalities quite difficult.

Likewise, X-ray phase-contrast imaging (also called phase-sensitive imaging) offers many advantages when compared to conventional attenuation imaging. In a sense, phase-contrast imaging, dark-field imaging and attenuation imaging are complementary imaging modalities, in which each modality can offer information about the imaged subject that is not revealed, or not revealed in the same detail, by the other modalities. For example, conventional X-ray attenuation imaging is particularly suitable for examining structures involving materials that cover a wide range of atomic number (Z). A typical example would be the detection of bone fractures, in which the high atomic number of calcium contrasts strongly with the relatively low atomic number of soft tissues. Phase-contrast imaging, on the other hand, is sensitive to variations in the refractive index, which depends on the electron density. Soft tissues, which typically consist of materials having very similar atomic number (e.g. due to carbon, hydrogen and oxygen being the predominant constituent elements), can nonetheless have widely varying electron densities, e.g. due to different molecular compounds.

Advantageously, these three, mutually complementary imaging modalities can be acquired in a single examination, e.g. by a single exposure (or at least in a single exposure sequence). Different processing algorithms can be applied to the same raw data to obtain, respectively, a differential phase-contrast image, a dark-field image and a conventional attenuation image.

The present invention relates to an insight of the inventors that certain imaging artefacts can be reduced or prevented by a specific use of radiation attenuating materials, which may, for example, be provided in the form of flexible sheets or wearable textiles.

Flexible radiation shielding in the form of sheets, aprons, gowns, curtains and the like may be used to protect a patient or hospital worker from radiation exposure. While radiation exposure is to some extent unavoidable when X-rays are used for imaging the body of the patient, or parts thereof, shielding materials can still be very useful to avoid or reduce exposure of a medical worker, when carrying out a procedure in the vicinity of the patient being imaged, to secondary radiation due to scattering on the body. Likewise, such materials may be used to cover parts of the body of the patient that do not need to be imaged, e.g. where such parts are particularly sensitive to radiation.

For example, US 2005/213712 discloses a radiation attenuation system for use in Computed Tomography (CT) procedures. A shield made of a radiation attenuating material is provided that can be used to block or attenuate radiation, i.e. to protect the patient and/or medical personnel. For example, CT fluoroscopy procedures are typically carried out to monitor an intervention while being carried out by medical personnel that is present in the scanner room, e.g. standing in close vicinity to the patient.

The radiation attenuation system of this example can include a wrap, a shield, a cloth, or a garment. Such garment can be used to cover portions of the patient during the CT procedure that are not going to be examined. The garment can include a fenestration area to provide access to a target area, e.g. such that this target area can be scanned, while the rest of the body is protected from radiation. However, such prior-art garments or other radiation shielding means are typically not used to improve image quality or to solve a problem that involves undesirable imaging artefacts. Therefore, whilst such radioprotective garment is known in the art, it is noted that the relevancy of such prior art for the presently disclosed invention might not be a priori obvious in view of the aim and purpose that underlies the present invention.

### SUMMARY OF THE INVENTION

It is an object of embodiments of the present invention to provide good and efficient means and methods to prevent or reduce a loss of image quality in X-ray dark-field imaging, e.g. to reduce or prevent image artefacts.

It is an advantage of embodiments of the present invention that artefacts in X-ray dark-field imaging can be reduced or avoided.

It is an advantage of embodiments of the present invention that artefacts in X-ray dark-field imaging due to crosstalk between pixels can be reduced or avoided. For example, in dark-field imaging, even for a small fraction of crosstalk, the absolute signal intensity in the directly irradiated detector part can be orders of magnitude larger than the intensity seen by diagnostically relevant detector regions. Thus, crosstalk can contribute substantially to the detected signal in the "shadow" of the patient.

It is an advantage of embodiments of the present invention that direct irradiation of detector elements by direct X-ray radiation from the X-ray source in an X-ray imaging apparatus (e.g. where the radiation is substantially unattenuated and/or unmodulated by any material between the source and detector), which would or could imply a substantial crosstalk effect between pixels, can be reduced or avoided.

It is an advantage of embodiments of the present invention that adverse effects due to deviation from linearity of the detector response can be avoided or reduced, e.g. such that correction and/or processing methods (e.g. a point spread function (PSF) deconvolution before applying a phase retrieval algorithm) that depend on a linear dose response of the detector are not required to compensate for crosstalk effects. It is to be noted that such processing that requires a linear response could fail to reach the intended result, e.g. potentially causing further imaging artefacts, when the detector response is not sufficiently linear.

For example, crosstalk can be corrected by a deconvolution with the detector's point spread function. However, this depends on a linear detector response, which is not guaranteed. Particularly, in directly irradiation detector regions saturation can occur, such that the detector response is no longer linear. It is an advantage of embodiments of the present invention, that crosstalk induced artefacts can be reduced or avoided, even if the detector response is not linear.

It is an advantage of methods and means in accordance with embodiments of the present invention that a patient can be provided with clothing that is suitable for use in an X-ray phase-contrast and/or dark-field imaging examination, e.g. thus avoiding discomfort due to nakedness. Nonetheless, this clothing does not adversely affect the image quality, e.g. as could be the case for X-ray scattering on wool or cotton fibers. Moreover, embodiments of the present invention can even improve the image quality by preventing or reducing artefacts.

It is an advantage of methods and means in accordance with embodiments of the present invention that a cheap and/or efficient approach to reduce imaging artefacts is provided.

It is a further advantage that an approach in accordance with embodiments of the present invention can be easily combined with (e.g. extended to) radiation protection of the patient and/or medical workers by also covering parts of the patient where imaging is not needed (and/or where shielding is desirable for protection) with attenuating material.

A device and method in accordance with embodiments of the present invention achieves the above objective.

In a first aspect, the present invention relates to a method for reducing image artefacts in dark-field and/or phase-contrast X-ray imaging of a subject. The method comprises positioning the subject to be imaged between an imaging detector and an X-ray source of an X-ray imaging apparatus that is suitable for dark-field and/or phase-contrast X-ray imaging, and positioning an X-ray attenuating material between the imaging detector and the X-ray source. The X-ray attenuating material is positioned such that direct exposure of the detector by radiation emitted by the source is reduced, e.g. preferably minimized, e.g. prevented. Direct exposure refers to the direct irradiation of the detector by X-rays emitted by the source without passing through matter (except, obviously, air or another gas or gas mixture in the environment), particularly without passing through solid or liquid matter. Specifically, direct exposure may refer to the X-rays, emitted by the source, reaching the detector without passing through the subject being imaged.

A method in accordance with embodiments of the present invention may comprise imaging the subject using the X-ray imaging apparatus after the positioning of the subject and the X-ray attenuating material, i.e. such as to obtain an X-ray dark-field and/or phase-contrast image(s) of the subject.

In a method in accordance with embodiments of the present invention, positioning the X-ray attenuating material may comprise positioning the X-ray attenuating material such that at least 80%, e.g. at least 90%, e.g. at least 95%, e.g. at least 99%, e.g. substantially all, of the detector pixels are in the umbra of the X-ray attenuating material and/or of the subject, as cast by radiation emitted by the X-ray source.

In a method in accordance with embodiments of the present invention, positioning the X-ray attenuating material may comprise positioning the X-ray attenuating material such that the umbra of the X-ray attenuating material on the detector overlaps less than 10%, e.g. overlaps less than 5%, e.g. overlaps less than 1%, e.g. does not overlap, with the umbra of the subject on the detector.

In a method in accordance with embodiments of the present invention, positioning the X-ray attenuating material may comprise placing a flexible sheet on top of or under the subject and/or suspending the flexible sheet in front of or behind the subject with respect to the direction from the X-ray source to the imaging detector, the flexible sheet being placed and/or suspended such that the peripheral radiopaque part is arranged adjacent to the subject when viewed as projected onto the imaging detector from the vantage point of the X-ray source. The flexible sheet may comprise a central radiotransparent part and a peripheral radiopaque part that comprises the X-ray attenuating material.

A method in accordance with embodiments of the present invention may comprise clothing the subject in a garment comprising a central radiotransparent part and a peripheral radiopaque part that comprises the X-ray attenuating material.

In a method in accordance with embodiments of the present invention, the step of clothing may comprise clothing the subject in a garment in accordance with embodiments of the present invention.

In a method in accordance with embodiments of the present invention, the step of clothing may comprise clothing the subject in radiotransparent clothing, e.g. a generic hospital gown or everyday clothes that do not interfere with dark-field and/or phase contrast imaging, and attaching one or more sheets of X-ray attenuating material to the radiotransparent clothing such as to extend away from the subject.

A method in accordance with embodiments of the present invention may comprise placing a cushion in (e.g. direct) contact with the subject, such that a deformable material of the cushion, comprising the X-ray attenuating material, abuts against the subject to conform to a contour of subject. For example, the cushion may be pushed against the side (or cushions may be pushed against each respectively side) of a patient, e.g. to the side(s) of a patient lying on an imaging examination couch. Thus, the deformable X-ray attenuating material of the cushion may form a radiopaque region adjacent to the subject, e.g. preferably without any significant gaps or openings in between.

A method in accordance with embodiments of the present invention may, additionally or alternatively, comprise placing the subject on a cushion that comprises the X - ray attenuating material, such that the weight of the subject displaces a deformable material of the cushion to reduce the thickness of the cushion underneath the subject to a (substantially) radiotransparent layer and to create a mass of deformable X-ray attenuating material bulging out at the sides of the subject, thus forming a radiopaque region adjacent to the subject.

In a second aspect, the present invention relates to a garment that comprises a central radiotransparent part and a peripheral radiopaque part, the peripheral radiopaque part comprising an X-ray attenuating material.

In a garment in accordance with embodiments of the present invention, the central radiotransparent part may be adapted for enclosing at least one body part of the subject when worn.

In a garment in accordance with embodiments of the present invention, the central radiotransparent part may be stretchable such as to tightly conform to contours of the at least one body part when worn.

A garment in accordance with embodiments of the present invention may be or may comprise a hospital gown.

In a garment in accordance with embodiments of the present invention, the peripheral radiopaque part may comprise the X-ray attenuating material in the form of a flexible material that is connected along at least part of its circumferential edge to a trunk section of the central radiotransparent part as well as to a sleeve of the garment, such that the flexible X-ray attenuating material forms a wing-like appendage between the trunk of the body and an arm when the garment is worn.

In a garment in accordance with embodiments of the present invention, the peripheral part and the central part may be detachable from and re-attachable to each other.

In a third aspect, the present invention relates to a flexible sheet of X-ray attenuating material that has a first edge and a second edge, adjacent to the first edge, in which the first edge and the second edge are at an angle in the range of 10° to 135° with respect to each other. The first edge and the second edge are provided with connectors to attach the first edge to a sleeve of a shirt or gown and to attaching the second edge to a trunk section of the shirt or gown, such as to obtain a garment in accordance with embodiments of the second aspect of the present invention when attached to the shirt or gown.

In a fourth aspect, the present invention relates to a cushion comprising an X-ray attenuating material. The cushion is deformable such as to conform to a contour (e.g. outer shape) of the subject when it is placed in abutment with the subject to form a mass of deformable X-ray attenuating material against a side of the subject.

The cushion may also (alternatively or additionally) be adapted to reduce the thickness of the cushion to a substantially radiotransparent layer underneath a subject, when the subject is placed upon the cushion, such that a mass of deformable X-ray attenuating material bulges out at the sides of the subject, when placed upon the cushion, to form a radiopaque region adjacent to the subject.

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

Fig. 1 shows an illustrative method in accordance with embodiments of the present invention.
Fig. 2 shows an illustrative garment in accordance with embodiments of the present invention.
Fig. 3 illustrates flexible sheets for use in a method in accordance with embodiments of the present invention.
Fig. 4 shows a head cap or mitten in accordance with embodiments of the present invention.
Fig. 5 shows a further illustrative garment in accordance with embodiments of the present invention.
Fig. 6 shows an illustrative cushion in accordance with embodiments of the present invention.
Fig. 7 shows a further illustrative cushion in accordance with embodiments of the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a method for reducing (or preventing; or avoiding) image artefacts in dark-field and/or phase-contrast X-ray imaging of a subject. In other words, the method may be intended to prevent or reduce a loss of image quality in X-ray dark-field and/or phase-contrast imaging that would otherwise result from pixel crosstalk. While applications of X-ray imaging commonly relate to the study of a person, e.g. of a patient's body for medical diagnostic purposes, the term 'subject', as used in the present description, is not necessarily limited to a human person. For example, the subject may be an animal, e.g. in veterinary applications, but could also include another living entity (e.g. plant matter) or even an inanimate object. In other words, 'subject' merely refers to the thing (or person) that is being studied by imaging, even though it may relate particularly to a human or animal body in accordance with some embodiments of the present invention. While many of the examples and/or embodiments described hereinbelow may assume explicitly or implicitly an animal subject, or even a human subject, it is to be noted that this does not imply that the same or other embodiments cannot relate to inanimate subjects, insofar reasonably conceivable.

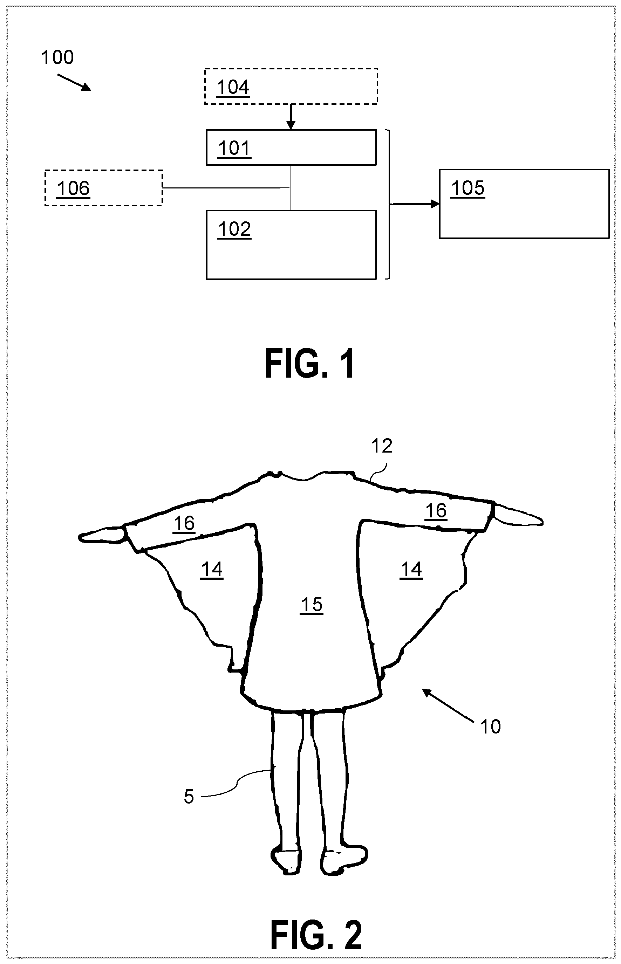

An illustrative method 100 in accordance with embodiments of the present invention in shown in Fig. 1.

The method 100 comprises positioning 101 the subject to be imaged between an imaging detector and an X-ray source of an X-ray imaging apparatus that is suitable for dark-field and/or phase-contrast X-ray imaging. Thus, in accordance with an imaging protocol and/or methodology as known in the art, the subject may be prepared for acquiring dark-field and/or phase-contrast images thereof.

The method 100 comprises positioning 102 an X-ray attenuating material between the imaging detector and the X-ray source, in which the X-ray attenuating material is positioned such that direct exposure of the detector by radiation emitted by the source is reduced, e.g. preferably minimized, e.g. prevented. Direct exposure refers to the direct irradiation of the detector by X-rays emitted by the source without passing through matter (except, obviously, air or another gas or gas mixture in the environment), particularly without passing through solid or liquid matter. Specifically, direct exposure may refer to the X-rays, emitted by the source, reaching the detector without passing through the subject being imaged.

The method may also comprise imaging 105 the subject using the X-ray imaging apparatus, e.g. after positioning 101,102 the subject and the X-ray attenuating material. For example, the X-ray source may be used to project X-ray radiation through the subject onto the detector, and the imaging detector may be used to acquire a projection image.

The X-ray attenuating material may be disposed in front of and/or behind the subject, e.g. when considering only its coordinate along (i.e. its projected position onto) the principal source-detector axis, or may be disposed at roughly the same distance to the source (and/or to the detector) as the subject.

It was observed by the inventors that avoiding direct exposure of detector pixels by rays that travel substantially unimpeded from the source to the detector can advantageously improve image quality. Thus, a loss of image quality in X-ray imaging, e.g. due to image artefacts, can be avoided or reduced. Direct irradiation of pixels can cause substantial crosstalk effects between detector pixels. While crosstalk is not limited to dark-field and phase-contrast imaging, the effect thereof can be particularly nefarious to image quality for these modalities, e.g. since dark-field and phase-contrast imaging, by their very nature, are sensitive to very small variations in pixel signal intensity. For example, the signal intensity in a directly irradiated detector region can be orders of magnitude larger than the intensity in the diagnostically relevant detector regions, such that crosstalk effects can become particularly pronounced. Furthermore, the processing applied to the raw detector pixel data may be sensitive to these crosstalk effects. Dark-field and phase-contrast imaging depend on further image processing to obtain the intended image, i.e. the dark-field and/or phase-contrast images are not directly observed by the detector. This processing may for example include an analysis of the phase stepping curve for each pixel location. However, this analysis may be disturbed by an additional background signal that propagated via the crosstalk effect from (e.g. peripheral) directly irradiated areas of the detector to the pixel of interest. Apart from the essential processing that may be required to obtain the dark-field and/or phase-contrast image, the image processing pipeline may also include other processing steps that, even though not strictly necessary, are desirable. For example, a point spread function (PSF) deconvolution may be applied before the phase retrieval algorithm is executed, but such deconvolution would also be sensitive to deviations from detector linearity.

Preferably, the X-ray attenuating material may be positioned such that at least 80%, even more preferred at least 90%, even more preferred at least 95%, or at least 98%, e.g. at least 99%, e.g. substantially 100%, of the detector pixels are in the umbra of the X-ray attenuating material and/or the subject with respect to radiation emitted by the source. Areas of overlap (or "underlap") between (shadows of) the subject and (of) the X-ray attenuating material may be minimized, e.g. avoided, such that the entire subject can be imaged at a high quality. Likewise, the X-ray attenuating material (or its shadow) may preferably conform (or adhere) well to the outer shape of the (shadow of) the subject, e.g. such as to leave no empty space between the X-ray attenuating material and the subject through which direct radiation exposure of the detector by X-rays from the source could adversely affect the image quality.

To reduce or avoid crosstalk effects, direct irradiation of detector pixels may preferably be reduced or avoided as much as possible or achievable. However, an overlap between the X-ray attenuating material and the subject (in the plane of the projected image) could obscure image details. This may be particularly undesirable in diagnostic medical imaging, where obscuring a lesion close to the body surface by the attenuating material could result in the lesion remaining undetected. This may be the case in, for example and without limitation, breast cancer or lymph node diagnosis. Therefore, it may be desirable in according with embodiments of the present invention to partition the detector area in a part where the illuminating rays pass through the subject being imaged and a part where the illuminating rays are attenuated by the X-ray attenuating material.

Even though the shape of the shadow cast by the attenuating material and the shape of the shadow cast by the subject onto the detector are preferably complementary, e.g. forming a partition of the active area of the detector, e.g. such that no overlap between these shadows is present, it is to be noted that, in particular applications, some overlap may be advantageous. However, the overlap, in such scenario, would be, preferably, deliberate and carefully planned to precisely determine the area or volume of the subject that is obscured by the attenuating material. For example, if only a part of the subject needs to be examined, or exposure of a particularly radiosensitive part of the subject is to be avoided, the X-ray attenuating material can also be used as radioprotective measure to block (or reduce) exposure of such radiosensitive part or part that is irrelevant for the intended purpose of the imaging. Thus, use of the X-ray attenuating material to cast a shadow onto a part of the detector that would otherwise be exposed to direct irradiation does not preclude the additional use of the X-ray attenuating material (or a further X-ray attenuating material; or a part of the X-ray attenuating material) for radioprotective purposes, e.g. to reduce the dose to the subject (or to reduce backscattering on the subject such that an operator or other worker in the vicinity of the subject receives less backscattered radiation).

The X-ray attenuating material may be provided in the form of a flexible sheet, e.g. a textile, cloth or fabric. Thus, the shape of the X-ray attenuating material can be easily deformed to conform to a contour (or contours) of the subject being imaged. However, other materials are not necessarily excluded. For example, a set of substantially rigid attenuator plates can be arranged such that it follows, at least to a sufficient degree, a contour of the subject, e.g. even though the individual plates might overlap in such approach. It will also be understood that the flexibility of a flexible sheet can be approximated by a sheet of rigid material that is partitioned by a large number of hinging connections, e.g. continuous or "piano" hinges, or independently slidable leaves of radiation attenuating material, e.g. similar in structure to a multi-leaf collimator as known for conformal beam shaping.

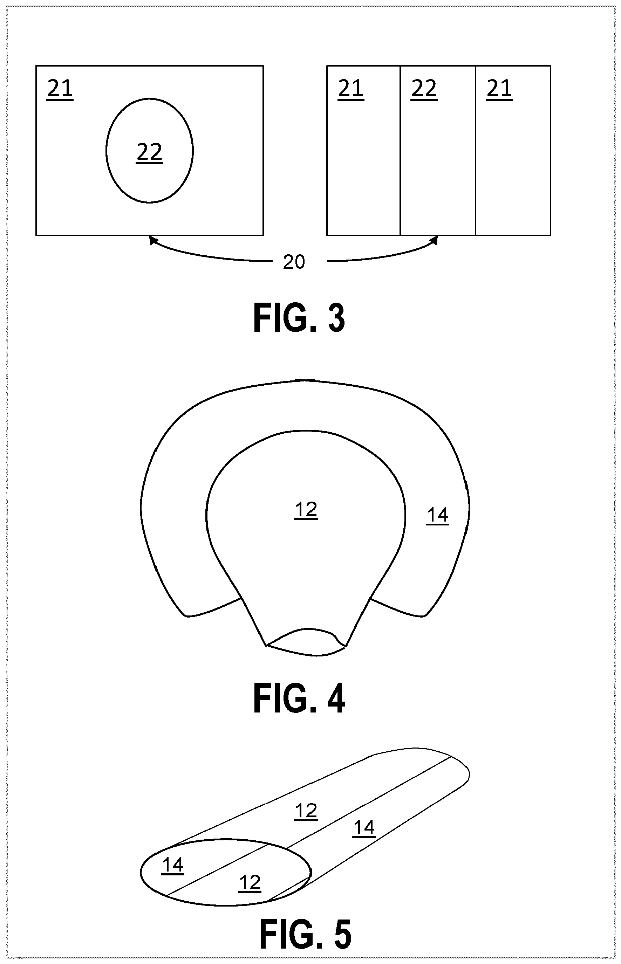

The X-ray attenuating material may be comprised in a flexible sheet, such as a textile, cloth or fabric, that comprises different materials and/or regions having different properties, e.g. a composite fabric or textile. For example, Fig. 3 shows two illustrative flexible sheets 20 in accordance with embodiments of the present invention. The flexible sheet 20 may comprise a central part 22, being central with respect to at least one one-dimensional direction, and at least one peripheral part 21, e.g. peripheral with respect to said one-dimensional direction. The central part 22 may be radiotransparent, e.g. may be adapted to allow X-ray radiation to pass through substantially unimpeded, and the peripheral part may comprise the X-ray attenuating material, e.g. may be adapted to block or strongly attenuate the X-ray radiation. The central part 22 is adapted for placing under or on top of the subject, e.g. over an area to be imaged. The peripheral part 21 is adapted to be arranged lateral to the subject (e.g. lateral with respect to the principal axis of the imaging system along which an X-ray beam is projected to image the subject).

Thus, positioning 102 the X-ray attenuating material may comprise placing the flexible sheet 20 over or under the subject (e.g. when positioned 101 for imaging), such that the central radiotransparent part 22 is over (or under) at least a part of the subject to be imaged, and such that an empty space (e.g. preferably all and any empty space) that falls in the field of view of the imaging setup is filling with the peripheral radiopaque part 21. For example, the subject may be lying on an imaging couch, and the flexible sheet 20 may be draped over the subject, e.g. such that the radiopaque part is supported by the couch and/or auxiliary support means in the vicinity. While possibly not as convenient, embodiments of the present invention do not exclude situations in which the subject is standing upright, e.g. in which case the flexible sheet can be supported by a freestanding support frame and/or the subject. For example, the sheet may be draped over shoulders of the subject, while the peripheral part may be held folded open by one or more suitable support elements, e.g. by attaching to or draping over an IV stand, tripod, or other suitable support. For example, the sheet may form a curtain, which (without limitation) may be supported by a rail or rail-like structure.

The flexible sheet may comprise connectors to connect the sheet to support elements, or may be permanently attached to (preferably adjustable) support elements. For example, such connectors may comprise clips, rings, laces, buckles, fasteners, Velcro elements and the like. Likewise, the connectors, or other (further) connectors, of the flexible sheet may be adapted to fixate the sheet (releasably) to the subject.

Thus, in use, the central part may be arranged on the subject, or a region of the subject to be imaged, such that the peripheral parts connected to the central part can block X-ray radiation passing around (or besides) this region of interest. Since the sheet is flexible, it can be folded together where needed, such that any free space beside the region of interest, e.g. next to the subject, is occupied by the attenuating material. The central part may comprise a material that has little impact on the X-ray radiation, e.g. not significantly attenuating but preferably also not significantly scattering X-ray radiation and/or not significantly altering the phase of the X-ray radiation. While reference is made to a central part 'of' the flexible sheet, it will be understood that this part may also be or may comprise a hole or holes to allow radiation to pass through the sheet. For example, the central part may be entirely void of material, or may comprise bands, straps or a loose lattice to leave as much space open as feasible.

While the order in which the X-ray attenuating material and the subject are positioned 101,102 between the source and detector can be reversed (i.e. can, in principle, be performed either way, or even simultaneously), it will be appreciated that it generally might be more convenient to position the X-ray attenuating material after the subject has been positioned, e.g. such as to cover a part of the detector with the attenuating material, wherein this part was left exposed to direct irradiation by the subject. In other words, another part of the detector may already be covered by the subject at this instant. It will be understood that it may be preferable to accurately position the subject in accordance with the requirements of the image to be acquired, and to position the attenuating material such as to cover the complement of the part of the detector that is already covered by the subject. Herein, the use of the term 'cover' is not intended to imply a direct contact with the detector (even though it is not necessarily excluded either), but merely refers to the object (e.g. resp. the subject or the attenuating material) being in an intermediate location between the source and detector such that the umbra of the object covers a part of the detector.

However, while positioning 102 the X-ray attenuating material may, preferably but not exclusively, be performed after positioning 101 the subject, in accordance with some embodiments of the present invention, the subject may be fitted with the X-ray attenuating material, e.g. may be clothed 104 by a garment 10 comprising the X-ray attenuating material, which may be (but not necessarily) performed before positioning 101 the subject to be imaged. Thus, the X-ray attenuating material may be implicitly positioned 102 as intended by positioning 101 the subject, since the subject was priorly fitted with the X-ray attenuating material, e.g. is wearing a garment comprising the X-ray attenuating material. Even if the X-ray attenuating material is not implicitly positioned appropriately, the act of positioning 102 the X-ray attenuating material may be conveniently reduced to merely arranging the X-ray attenuating material, e.g. draping (parts of) the garment, appropriately and/or verifying that the X-ray attenuating material is covering the intended area of the detector. Therefore, it will be appreciated that integrating the X-ray attenuating material in a garment may advantageously contribute to an efficient workflow, for example by reducing the required action to position the X-ray attenuating material to merely minor adjustments if and when needed.

Thus, the method 100 may comprise clothing 104 (i.e. dressing) the subject (e.g. particularly in case of the subject being an animal or human) in a garment that comprises the X-ray attenuating material. The subject may put on the garment, or may be clothed by a worker, or may be assisted by a worker in putting on the garment. Thus, the subject is provided with the garment such as to be worn by the subject, e.g. in a manner as is typical for clothing of its particular type.

It is noted that, typically, a dark-field and/or phase-contrast imaging examination would require the subject to undress and/or dress in a garment that is specifically suitable for use in such imaging examination. Therefore, the step of clothing 104 does not need to interfere substantially with a typical workflow, e.g. in a radiology department of a hospital.

Fig. 2 shows an illustrative garment 10 in accordance with embodiments of the present invention. In a second aspect, the present invention relates to such a garment 10. For example, the garment 10 may be suitable for being worn by the subject 5. Embodiments of the second aspect of the present invention may relate also to a flexible sheet 20 as described hereinabove, e.g. as shown in Fig. 3. For example, the garment may be a flexible sheet for draping on or around the subject. For example, such flexible sheet may comprise (even though not necessarily) a hole through which the head of the subject can be extended, e.g. the flexible sheet may be a poncho.

Like the flexible sheet, the garment 10 comprises a central radiotransparent part 12 and a peripheral radiopaque part 14, the latter comprising or consisting of the X-ray attenuating material. It will be understood that radiotransparent and radiopaque refer to the ability to resp. transmit and block ionizing radiation, particularly radiation in the spectrum used for the imaging procedure, i.e. X-ray radiation. It will also be understood that this refers to substantially transmitting and substantially blocking, e.g. within limits of practicality. For example, the radiotransparent part may preferably transmit as much (X-ray) radiation as achievable or practical and/or may block and/or scatter only as much as would be negligible or irrelevant for the imaging procedure, while the radiopaque part may preferably block as much (X-ray) radiation as achievable or practical and/or may transmit only as much as would negligible or irrelevant for the imaging procedure.

The central part 12 may be adapted to encircle or enclose one or more body parts. For example, the central part may comprise one or more tubular structures for wearing around the trunk, the neck, the head, and or the extremities. For example, a first tubular part may be suitable for wearing around the trunk, while a second and third tubular part may be suitable for wearing around respectively each arm, e.g. may be sleeves. It will be understood that, as is known for clothing, the tubular parts may be adapted for opening (e.g. using laces, zippers or other such means), or may even be open, e.g. may form into a (roughly) cylindrical shape only when being worn.

Thus, in a particularly advantageous approach, the X-ray attenuating material may be, or may be comprised in, an article of clothing, e.g. in a garment, such as a hospital gown (patient gown). As known in the art, such hospital gown may typically be a long loose piece of clothing worn by someone undergoing a medical procedure, i.e. undergoing a diagnostic imaging examination using phase-contrast or dark-field imaging. While such hospital gown is typically a long and loose piece of clothing, embodiments are not limited to the conventional shape or style of such gowns, e.g. the gown may be tight fitting and/or short. Particularly, in accordance with embodiments of the present invention, a tight fitting of the gown could even be preferred, as will be explained further hereinbelow. Other types of garment or clothing are not necessarily excluded either. For example, the garment may be implemented as a cap or balaclava, e.g. for use in a specific examination of the head and/or cervix in which the field of view of the imaging protocol may be limited to the head and/or neck region. Similarly, for a specific examination of a leg, a foot, the inguinal region, etc., the garment may be implemented as a sock, a single pants leg, pants, underpants, or the like. Likewise, the X-ray attenuating material may be implemented by (e.g. may comprise, consist of or be comprised in) a glove, a sleeve, a shirt, a short sleeve shirt, a long sleeve shirt, a sleeveless shirt, an undershirt, a bodysuit, or other types of clothing.

The garment 10 comprises a central radiotransparent part 12 and a peripheral radiopaque part 14, the latter comprising or consisting of the X-ray attenuating material. As was mentioned for the flexible sheet described hereinabove, 'central' may refer to being central only in the sense of a single direction, e.g. as illustrated by the flag-like structure shown in Fig. 3 (right). However, central may also refer to being central in the sense of more than one direction (e.g. Fig. 3, left). 'Central' does not require being at the geometrical or mathematical center, e.g. only refers to being more central than the peripheral part(s).

It will be understood that radiotransparent and radiopaque refer to the ability to resp. transmit and block ionizing radiation, particularly radiation in the spectrum used for the imaging procedure, i.e. X-ray radiation. It will also be understood that this refers to substantially transmitting and substantially blocking, e.g. within limits of practicality. For example, the radiotransparent part may preferably transmit as much (X-ray) radiation as achievable or practical and/or may block and/or scatter only as much as would be negligible or irrelevant for the imaging procedure, while the radiopaque part may preferably block as much (X-ray) radiation as achievable or practical and/or may transmit only as much as would negligible or irrelevant for the imaging procedure.

It is to be noted that having the subject wear normal (generic) clothing is generally not desirable in an X-ray dark-field or phase-contrast imaging examination. While the attenuative properties of clothing are typically not problematic in conventional X-ray attenuation imaging, textile fibers, such as cotton and/or wool, may scatter X-ray radiation quite a lot, such that dark-field and phase-contrast imaging would be adversely affected. Therefore, in a typical approach, a patient may be imaged without clothing. Nonetheless, this may cause some discomfort, such that it is a further advantage of methods and means in accordance with embodiments of the present invention that a patient can be provided with clothing that is suitable for use in an X-ray phase-contrast and/or dark-field imaging examination, i.e. avoiding discomfort due to nakedness while not adversely affecting the image quality. Moreover, embodiments of the present invention even aim at improving the image quality by preventing or reducing artefacts due to pixel crosstalk. For example, for increased comfort, the radiotransparent part may be substantially transparent (and non-scattering) to X-ray radiation, while it may be opaque, translucent or otherwise at least obscuring to some extent for optical wavelengths. However, embodiments are not necessarily limited thereto. For example, to avoid any interference of the radiotransparent part with the imaging procedure, the radiotransparent part may comprise one or more (open) windows to let radiation pass through unimpeded. In extremis, the radiotransparent part may comprise or consist of straps, bands, belts, webbing or other elongate elements forming a holster, suspenders, or a similar structure for supporting the radiopaque part. For example, the radiopaque part may be (preferably firmly) held in place, at a desired position and/or orientation on the body, by one or more of such straps, bands, belts, webbing or other such elements, such that the 'radiotransparent part' may consist of merely the absence of material between such supporting elements and/or the supporting elements themselves. While the supporting elements may, e.g. preferably, be composed of a radiotransparent material, it will be understood that this is not strictly required, e.g. the influence of such supporting elements on the acquired images (and particularly on the quality thereof) may be reduced by carefully designing and positioning such supporting elements.

The peripheral radiopaque part 14 of the garment 10 may comprise the X-ray attenuating material in the form of a flexible material that is connected along at least part of its circumferential edge to a trunk section 15 of the central radiotransparent part 12 and a sleeve 16, e.g. an arm section that may also form part of the central radiotransparent part 12. The garment may comprise a trunk section 15 and two sleeves 16 (arm sections), for respectively being worn around the subject's trunk, left arm and right arm. Thus, as shown in Fig. 2, the peripheral radiopaque part may comprise a left wing supported by the left arm and the (left side of the) trunk and a right wing supported by the right arm and the (right side of the) trunk. In other words, the left and right wing may form batwing-like appendages to the central part, which may, on its own, form (without limitation) a hospital gown or similar garment.

The (or each) peripheral part 14 and the central part 12 may be detachable from and re-attachable to each other. For example, the left and/or right wing may be connectable and detachable to the trunk section and/or sleeve. For example, the central part 12 may comprise or consist of a conventional hospital gown, or generic piece(s) of clothing, as readily available. The peripheral part 14 may comprise one or more pieces of X-ray attenuating flexible material that is adapted for being clipped onto (or otherwise connected to) the trunk section and sleeve of the hospital gown or other pieces of clothing.

The method 100 may comprise clothing 104 the subject in a garment 10 in accordance with embodiments of the present invention, in which the garment 10 is purposely designed for this purpose. However, the method may also comprise clothing the subject in generic clothing, in a readily available hospital gown, or in a gown specifically designed to avoid interference with dark-field and/or phase-contrast imaging. Positioning 102 the X-ray attenuating material between the imaging detector and the X-ray source in the method 100 may thus comprise attaching a piece (e.g. sheet) of X-ray attenuating flexible material between one arm and a trunk section of the clothing, e.g. and possibly repeating this with another such sheet for the other arm.

Therefore, embodiments of the second aspect of the present invention may also relate to a kit of parts, comprising a radiotransparent garment and at least one sheet of flexible X-ray attenuating material that is attachable to (and detachable from) the garment, e.g. to a trunk section and an arm section, e.g. sleeve, of the garment to form wing-shaped appendages between the subject's trunk and arm(s) when worn.

Embodiments of the second aspect of the present invention may also relate to a flexible sheet of X-ray attenuating material having a first and a second edge, e.g. at an angle in the range of 10° to 135° with respect to each other, preferably at an angle in the range of 45° to 100°, in which the first and second edge are provided with connectors (attachment elements), such as clips, rings, laces, buckles, fasteners, zippers, Velcro elements and the like. Thus, the sheet of X-ray attenuating material is adapted for being (releasably) attached to a garment between a trunk section and an arm section of the garment. Without limitation, the sheet may be triangular or roughly triangular. Embodiments may also relate to a pair of such sheets, e.g. for attaching to respectively the left sleeve and the right sleeve of a garment.

It will be understood that the embodiments explained hereinabove can be modified in a straightforward manner for the purpose of imaging specific body parts, such as bone imaging of an extremity and/or the head and/or neck. In such case, the garment may comprise or consist of a sleeve dimensioned to encompass the body part of interest and at least one (e.g. preferably at least two: e.g. preferably two) flexible sheet of X-ray attenuating material that is attached or attachable to the sleeve, e.g. along an edge of the flexible sheet. For example, two such flexible sheets may be attached or attachable to opposite sides of the sleeve. Furthermore, in accordance with some embodiments, the sleeve may have a closed end, e.g. to form a glove or head cap. It will be understood that, for such case, also a single flexible sheet may be attached or attachable to opposite sides of the sleeve, by following a single contour line that extends around the closed end. An example is illustrated in Fig. 4.

It will also be understood that the central radiotransparent part 12 of the garment, e.g. the sleeve, glove, cap, or other item for generally encompassing a body part when worn, may be flexible and stretchable such as to conform well to the contours of the body part when worn. The peripheral radiopaque part 14 may be attached (or attachable) to the central part 12, e.g. along an edge of the peripheral radiopaque part, such that, when worn, the bodily contours can be precisely followed. Thus, advantageously, direct exposure of the detector through a gap between the subject and the X-ray attenuating material can be avoided, while obscuring of details of the subject's anatomy by the X-ray attenuating material can also be easily avoided by ensuring that the peripheral part is, in use, extended away from the subject and preferably in a direction (close to) parallel to the imaging plane.

The examples given hereinabove relate to the central part 12 comprising one or more tubular sleeves forming a garment, e.g. joined in a T-shape as shown in Fig. 2 to form a shirt or gown, or to the central part 12 being shaped as a cap, as shown in Fig. 4. However, embodiments are not necessarily limited thereto. In these examples, the peripheral part(s) 14 may be attached or attachable to the central part along an edge or edges (e.g. Fig. 2) of the peripheral part (without necessarily being limited to being joined at the edge or edges).

However, other embodiments can be envisioned. For example, Fig. 5 shows a garment, in this example a sleeve, in which the central radiotransparent part 12 and the peripheral radiopaque part 14 form different angular regions (arcs) of the composite sleeve. When worn, the radiopaque parts may be positioned to the sides of the body part being imaged (when viewed in the imaging plane). Thus, the projection of the radiopaque parts in the imaging plane will form wide bands to either side of the body part being imaged. It will be understood that, in this example, the garment may preferably be loosely fitting and/or no relevant features to be imaged may be present near the surface of the body part (e.g. where it might be obscured by the radiopaque parts. For example (without limitation), such approach could be useful for specifically imaging a long bone, e.g. the femur.

It will also be understood that the radiotransparent part 12 and the radiopaque part 14 may gradually transition, e.g. by varying a density of a radiation absorbing material included in the garment's principal material from substantially zero (in the center of the radiotransparent part) to a predetermined maximum (in the center of the radiopaque part). In other words, in accordance with some embodiments, the radiotransparent part and the radiopaque part may not be defined by a clear line separating the different materials, yet remain clearly detectable as a whole by their radiation attenuating properties nonetheless.

The X-ray attenuating material in accordance with embodiments of the present invention may be similar to materials known in the art for radioprotective curtains, aprons and the like. For example, the X-ray attenuating material may comprise a carrier material, such as a polymer, a foam and/or a rubber, that is loaded with powdered lead (Pb), tungsten (W), cadmium (Cd), indium (In), tin (Sn), antimony (Sb), cesium (Cs), barium (Ba), cerium (Ce), gadolinium (Gd), gold (Au), silver (Ag), bismuth (Bi), and/or alloys thereof and/or combinations thereof. For example, a high Z (atomic number) material, e.g. a metal, may be embedded in a rubber or polymer matrix to obtain X-ray attenuating properties while still providing (preferably) a flexible material. It is an advantage of incorporating the attenuating material as a powder (e.g. micro- or nano-particles) that a good composite material can be achieved even for choices of attenuating material that may be quite brittle, such as tungsten, or have other mechanical properties that are not ideal for the intended purpose.

Other approaches than embedding a powdered material may also be envisioned, e.g. by applying a coating with a suitable material (e.g. see the examples given hereinabove) to a fiber or fiber-like material that can be woven into a textile or can be used to form a textile, cloth, fabric or similar material (e.g. by any suitable method, such as weaving, knitting, melting, bonding etc.). As another example, a plurality of foils of high-Z materials, e.g. metal foils, may be combined in a multi-layer (e.g. sandwich) structure, e.g. by alternating layers of the attenuating material and of a structural carrier that provides integrity as well as (preferably) flexibility.

The X-ray attenuating material may have a lead-equivalent thickness in the range of 100 µm to 5 mm, e.g. in the range of 150 µm to 500 µm. Clearly, an upper limit to the Pb-equivalent thickness is only imposed by practical considerations, such as cost, weight and flexibility of the material. The lower limit may be selected such as to achieve at least 80%, preferably at least 90%, even more preferred at least 95%, e.g. at least 99%, attenuation of the X-ray beam used for imaging. Even though the relevant beam quality would be a photon beam of the spectrum used in the imaging examination at hand, for the purpose of clarity and definiteness, the lead-equivalent thickness and/or percentage of beam attenuation can be measured in accordance with standard test setups, methodologies and standard beam qualities, such as the IEC RQR 6 standard beam quality (see e.g. IEC 61267, e.g. of 2005).

As a further illustrative example, the X-ray attenuating material may be provided in the form of a flexible cushion, e.g. a pillow, mattress or element of a couch, or may be comprised in such cushion. For example, positioning 102 the X-ray attenuating material and positioning 101 the subject may comprise placing 106 a cushion 30 in (e.g. direct) contact with the subject, such that a deformable material of the cushion, comprising the X-ray attenuating material, abuts against the subject to conform to the shape of a contour of the subject. For example, the cushion (or cushions) may be pushed against the side (or sides) of a patient. Thus, the deformable X-ray attenuating material of the cushion may form a radiopaque region adjacent to the subject, e.g. preferably without leaving any (significant) gap or opening in between the cushion and the subject.

The cushion 30 comprises a deformable X-ray attenuating material, e.g. as a (e.g. component of) a gel, a foam, a memory foam, a liquid, non-Newtonian liquid and/or a powder.

Alternatively, or additionally, the cushion may comprise an inflatable part, e.g. an inflatable balloon or bladder, or a plurality of such inflatable parts. For example, the inflatable part or parts may be inflated, in use of the cushion, to adapt to the shape of the subject (or at least in the region where the subject is contacted by the cushion in use thereof). By including separately inflatable parts, the shape of the subject may be followed even better, e.g. by inflating each part up to a pressure that achieves the best (or a good) result. The material forming the (wall of the) inflatable part or parts may be elastic, e.g. stretchable, such that the shape can deform well by inflation (even though less elastic materials are not necessarily excluded). The X-ray attenuating material may cover the inflatable part(s), e.g. may comprise a flexible sheet that can be deformed by inflating the inflatable part to follow the contour of the subject (in at least the relevant region). The X-ray attenuating material may also be comprised in the inflatable part(s), e.g. in the wall thereof. For example, the X-ray attenuating material may be formed by a coating on (the wall of) the inflatable part(s), e.g. by a high-Z (high atomic number) metallic coating or other coating suitable for attenuating X-ray radiation.

The cushion 30 may comprise a strap, a belt, a cord, or similar fixation means, to hold the cushion in place against the subject, e.g. by strapping the cushion to the subject.

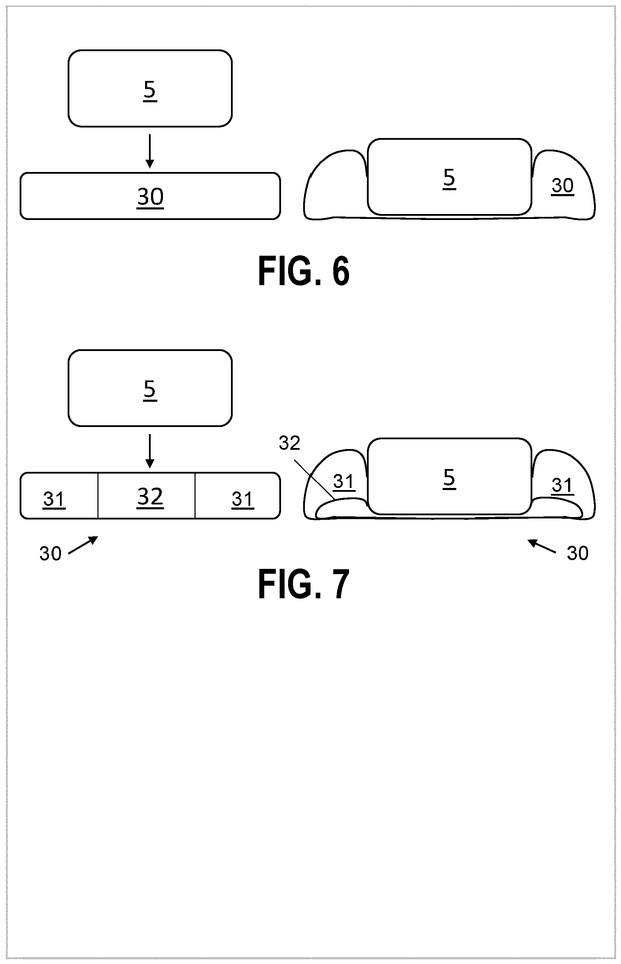

Placing 106 the cushion 30 in contact with the subject 5 may also comprise placing 106 the subject 5 on the cushion 30, e.g. as shown in Fig. 6. Thus, the weight of the subject may displace the deformable material, such that only a thin layer remains underneath the subject. In contrast, a large mass of the deformable X-ray attenuating material, displaced by the weight of the subject, may bulge out at the sides of the subject, thus forming a thick accumulation of X-ray attenuating material at the sides of the subject. It is an advantage that thus a good barrier to X-ray radiation is formed that conforms well to the contours of the subject, while only a small attenuating mass may remain present underneath the subject. Furthermore, the cushion may comprise different regions (sub-volumes) that comprise different materials. For example, a central radiotransparent part 32 of the cushion may be filled with a material that does not scatter or attenuate X-rays to a non-negligible extent, while a peripheral radiopaque part(s) 31 may comprise (or consist of) the X-ray attenuating material, e.g. as illustrated in Fig. 7. Thus, the impact on image quality of any material underneath the subject that is not entirely displaced by placing the subject on the cushion is further reduced. The central radiotransparent part may comprise or consist of a material having suitable mechanical properties, e.g. a high deformability, elasticity and/or flexibility. The radiopaque part may have similar mechanical properties, but substantially different properties with respect to interactions with ionizing radiation. This approach also has the additional benefit of providing stabilization to the subject, e.g. providing additional comfort and/or preventing or reducing movement artefacts.

## Claims

1. A method (100) for reducing image artefacts in dark-field and/or phase-contrast X-ray imaging of a subject, said method comprising:
- positioning (101) the subject to be imaged between an imaging detector and an X-ray source of an X-ray imaging apparatus, and
- positioning (102) an X-ray attenuating material between the imaging detector and the X-ray source such that direct exposure of the detector by radiation emitted by the source during imaging of the subject is reduced or prevented.

2. The method of claim 1, comprising imaging (105) the subject using the X-ray imaging apparatus after said positioning (101,102) of the subject and the X-ray attenuating material.

3. The method of any of the previous claims, wherein positioning (102) said X-ray attenuating material comprises positioning the X-ray attenuating material such that at least 80% of the detector pixels are in the umbra of the X-ray attenuating material and/or of the subject for radiation emitted by the X-ray source.

4. The method of claim 3, wherein positioning (102) said X-ray attenuating material comprises positioning the X-ray attenuating material such that the umbra of the X-ray attenuating material on the detector overlaps less than 10% with the umbra of the subject on the detector.

5. The method of any of the previous claims, wherein positioning (102) said X-ray attenuating material comprises placing a flexible sheet (20) on top of or under the subject and/or suspending the flexible sheet (20) in front of or behind the subject with respect to the X-ray source to imaging detector direction, wherein said flexible sheet comprises a central radiotransparent part (22) and a peripheral radiopaque part (21) that comprises the X-ray attenuating material, such that the peripheral radiopaque part (21) is arranged adjacent to the subject when viewed as projected onto the imaging detector from the vantage point of the X-ray source.

6. The method of any of the claims 1 to 4, comprising clothing (104) the subject in a garment (10) comprising a central radiotransparent part (12) and a peripheral radiopaque part (14) that comprises the X-ray attenuating material.

7. The method of claim 6, wherein said clothing (104) comprises clothing the subject in the garment (10) in accordance with any of the claims 9 to 13, or wherein said clothing (104) comprises clothing the subject in radiotransparent clothing and attaching one or more sheets of X-ray attenuating material to said radiotransparent clothing such that the one or more sheets extend away from the subject.

8. The method of any of the claims 1 to 4, comprising placing (106) a cushion (30) in contact with the subject, such that a deformable material of the cushion, comprising the X-ray attenuating material, abuts against the subject such as to conform to a contour of subj ect.

9. A garment (10) comprising a central radiotransparent part (12) and a peripheral radiopaque part (14) that comprises an X-ray attenuating material.

10. The garment of claim 9, wherein said central radiotransparent part (12) is adapted for enclosing one or more body parts of the subject when worn.

11. The garment of claim 9 or 10, wherein said central radiotransparent part (12) is stretchable such as to tightly conform to contours of the at least one body part when worn.

12. The garment of any of the claims 9 to 11, wherein said peripheral radiopaque part (14) comprises the X-ray attenuating material in the form of a flexible material that is connected along at least part of its circumferential edge to a trunk section (15) of the central radiotransparent part (12) as well as to a sleeve (16) of the garment, such that the flexible X-ray attenuating material forms a wing-like appendage between the trunk and an arm when the garment is worn.

13. The garment of any of the claims 9 to 12, wherein said peripheral part (14) and said central part (12) are detachable from and re-attachable to each other.

14. A flexible sheet of X-ray attenuating material having a first edge and a second edge, adjacent to the first edge, wherein said first edge and said second edge are at an angle in the range of 10° to 135° with respect to each other, wherein said first edge and said second edge are provided with connectors for attaching the first edge to a sleeve of a shirt or gown and for attaching the second edge to a trunk section of the shirt or gown, such as to obtain a garment in accordance with any of the claims 9 to 13 by said attaching.

15. A cushion (30) comprising an X-ray attenuating material, said cushion being deformable such as to conform to a contour of the subject when it is placed in abutment with the subject to form a mass of deformable X-ray attenuating material against a side of the subj ect.
